# EUROPÄISCHE PATENTANMELDUNG

(11) **EP 3 326 691 A1**
(43) Veröffentlichungstag der Anmeldung: **30.05.2018**
(21) Anmeldenummer: 16200256.2
(22) Anmeldetag: 23.11.2016
(51) Int. Cl.: A61N 1/375, A61N 1/39, A61N 1/372

(54) **MEDIZINISCHES IMPLANTAT UND SYSTEME ZUR INTRAVASKULÄREN IMPLANTATION**

(71) Anmelder: BIOTRONIK SE & Co. KG, 12359 Berlin (DE)
(72) Erfinder: Bitzer, Andreas, 8032 Zürich (CH)
(74) Vertreter: Galander, Marcus

(57) **Zusammenfassung**

Die Erfindung beschreibt ein medizinisches Implantat zur intravaskulären Implantation, aufweisend ein Implantatgehäuse und mindestens ein erstes und ein zweites Beförderungselement, wobei das erste und das zweite Beförderungselement in einer Ebene am Implantatgehäuse angeordnet sind, wobei das erste Beförderungselement drehbar um eine erste Rotationsachse gelagert ist und das zweite Beförderungselement drehbar um eine zweite Rotationsachse gelagert ist.

## Beschreibung

Die Erfindung betrifft ein medizinisches Implantat, insbesondere im Herzkreislaufsystem implantierte, miniaturisierte Implantate, sowie Systeme zur intravaskulären Implantation derselben.

Ein Beispiel für derartige Implantate stellen implantierbare Drucksensoreinrichtungen dar, insbesondere eine Miniaturdrucksensoreinrichtung, die in einem tierischen oder menschlichen Körper implantierbar ist. Drucksensorvorrichtungen einschließlich mikroelektromechanischer Systeme (MEMS) und dazugehörige Chipsysteme sind in der Technik bekannt. Beispielsweise werden Drucksensorvorrichtungen mit MEMS-Chips für elektrische Implantate zur Verbesserung der Herzfunktion eines Patienten eingesetzt. Für derartige Zwecke einsetzbare Chipsysteme müssen einerseits hinreichend präzise Messergebnisse liefern, andererseits sehr kleine Abmessungen aufweisen, sodass sie problemlos implantierbar sind und die physiologische Aktivität des Organismus nicht einschränken, beispielsweise für Blutdruckmessungen in der Pulmonalarterie.

Ein anderes Beispiel für ein solches Implantat sind Schrittmacherimplantate, welche für die Implantation intravaskulär befördert werden, um schließlich in einem Blutgefäß implantiert zu werden, wie zum Beispiel miniaturisierte Herzschrittmacher ohne lange Elektrodenleitungen (sogenannte 'Leadless' Schrittmacher).

Eine technische Herausforderung im Zusammenhang mit solchen Implantaten stellt die Beförderung des Implantats durch das Herzkreislaufsystem dar. Trotz Miniaturisierung der Implantatabmessungen kann es dabei Schwierigkeiten bei der Beförderung des Implantats durch das Herzkreislaufsystem geben, insbesondere wenn das Implantat durch enge, kurvenreiche Blutgefäße mit kleinem Kurvenradius gebracht werden muss. Ein zweites Problem stellt die Platzierung und zuverlässige Fixierung des Implantats am Implantationsort, d. h. im Kontakt mit dem Blutstrom, dar. Erforderlich ist dafür eine ortsfeste Fixierung des Implantats, ohne dass der Blutfluss maßgeblich gestört wird. Wünschenswert sind ein unkompliziertes Implantationssystem und -verfahren sowie eine einfache Möglichkeit zur Explantation.

US 2016/0082270 A1 beschreibt eine Fixiereinheit, welche ausgestaltet ist, ein medizinisches Implantat im Patienten zu verankern.

Bekannt aus dem Stand der Technik sind Systeme basierend auf Schleifen aus Draht, welche an den Seiten des Implantats angebracht sind und das Implantat in einem Blutgefäß halten. Derartige Systeme weisen jedoch mehrere Nachteile auf: Die Schleifen aus Draht werden im implantierten Zustand relativ stark gekrümmt und somit das Material stark beansprucht. Des Weiteren nehmen die Drahtschleifen einen relativ großen Raum ein und die Verbindung der Schleifen zum Gehäuse, welche gleichzeitig eine elektrische Durchführung darstellt, ist in der Herstellung aufwändig.

Stent-basierte Lösungen für die Befestigung von Implantaten im Blutgefäß sind ebenfalls bekannt. Stent-artige Lösungen haben den großen Nachteil, dass sie relativ steif und somit schwer durch Blutgefäße zu führen sind. Zudem neigen Stent-Systeme zum Migrieren, d. h. es besteht die Möglichkeit, dass das Implantat auf Dauer nicht am Implantationsort verbleibt, sondern im Blutgefäß 'wandert'. Auch sind Stent-Systeme und zugehörige Implantationssysteme aufwändig und kostenintensiv in der Herstellung.

Es ist deshalb Aufgabe der vorliegenden Erfindung, ein medizinisches Implantat bereitzustellen, das durch einfache Weise durch gekrümmte Blutgefäße des Herzkreislaufsystems befördert werden kann. Eine weitere Aufgabe der vorliegenden Erfindung liegt in der Bereitstellung eines medizinischen Implantats, das im Herzkreislaufsystem dauerhaft und ortsfest implantiert werden kann. Auch kann eine weitere Aufgabe darin gesehen werden, ein medizinisches Implantat bereitzustellen, welches auf einfache Art im- und explantiert werden kann.

Auch ist es Aufgabe der vorliegenden Erfindung, Systeme zur Implantation eines besagten medizinischen Implantats bereitzustellen, die über einen unkomplizierten Aufbau verfügen und darüber hinaus einfach in der Verwendung und in der Herstellung sind.

Eine weitere Aufgabe kann darin gesehen werden, ein medizinisches Implantat bereitzustellen, das nach der Implantation im Herzkreislaufsystem den Blutfluss nicht maßgeblich beeinträchtigt bzw. behindert.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird gemäß der vorliegenden Erfindung ein medizinisches Implantat zur intravaskulären Implantation vorgeschlagen, das ein Implantatgehäuse und mindestens ein erstes und ein zweites Beförderungselement aufweist, welche in einer Ebene am Implantatgehäuse angeordnet sind. Dabei ist das erste Beförderungselement drehbar um eine erste Rotationsachse gelagert, und das zweite Beförderungselement drehbar um eine zweite Rotationsachse gelagert.

Im Zusammenhang mit der vorliegenden Erfindung sind jegliche Arten von medizinischen Implantaten denkbar, die intravaskulär implantiert werden und/oder beim Implantationsvorgang durch Blutgefäße befördert werden müssen. Beispiele hierfür sind kardiale Implantate wie beispielsweise Schrittmacher, insbesondere Schrittmacherimplantate ohne Elektrodenleitungen (sogenannte 'leadless'-Implantate) zur Implantation in einer Herzkammer, kraniale Implantate, Stent- oder Coil-Implantate o. Ä.. Eine Ausführungsform sieht als medizinisches Implantat einen miniaturisierten Drucksensor zur Implantation in der Pulmonalarterie vor, um invasive Blutdruckmessungen in einem längeren Zeitraum durchführen zu können.

Das Implantatgehäuse besteht vorzugsweise aus Material, welches Biokompatibilität aufweist und gleichzeitig robust gegenüber den Einflüssen im Umgebungsmilieu des Implantationsortes ist. Ein geeignetes Material ist beispielsweise Titan.

Die Gehäusegeometrie ist vorzugsweise derart gewählt, dass eine Beförderung durch bzw. Implantation in einem Blutgefäß auf einfache Art möglich ist.

Im Sinne der Erfindung ist unter einem Beförderungselement eine Komponente zu verstehen, die dazu dienlich ist, die Beförderung des Implantats durch Blutgefäße zum Implantationsort zu vereinfachen. Erfindungsgemäß sollen die Beförderungselemente drehbar gelagert sein.

Gemäß einer vorteilhaften Ausführungsform der vorliegenden Erfindung weist das Implantatgehäuse eine Form mit einem proximalen und einem distalen Ende auf, wobei das erste Beförderungselement nahe dem proximalen Ende und das zweite Beförderungselement nahe dem distalen Ende angeordnet ist. Dabei sind in einer bevorzugten Ausgestaltung der vorliegenden Erfindung das erste und das zweite Beförderungselement an mindestens einer seitlichen Außenfläche des Implantatgehäuses angeordnet.

Eine geeignete geometrische Grundform für das Außengehäuse stellt beispielsweise ein Quader dar; auch denkbar wäre ein Zylinder, ein Ellipsoid, oder jegliche stabähnliche oder längliche Form. Vorteilhaft wären hierbei Formen mit abgerundeten Ecken und Kanten, um den Strömungswiderstand zu verringern, sodass der Implantationsvorgang vereinfacht und die Thrombenbildung am Implantat erschwert wird. Die Form kann definierte ebene Außenflächen aufweisen, wie es bei einem Quader der Fall ist, sowie gewölbte Außenflächen, wie bei einem Ellipsoid oder der Mantelfläche eines Zylinders. Besitzt das medizinische Implantat beispielsweise eine quaderförmige Außenform, so können die Beförderungselemente an einer seitlichen Außenwand angeordnet sein, wobei das erste Beförderungselement nahe dem proximalen Ende des Implantatgehäuses, und das zweite Beförderungselement nahe dem distalen Ende des Implantatgehäuses angeordnet ist. Dabei können die Beförderungselemente durch Teile des Implantatgehäuses gebildet werden oder mit dem Implantatgehäuse verbunden sein mittels einer Schraub-, Schweiß-, Löt-, Klebeverbindung o. Ä.. Ist das Implantatgehäuse zylinderförmig mit einer Mantelfläche und zwei Grundflächen, so können die mindestens zwei Beförderungselemente entlang einer Linie an der Mantelfläche angeordnet sein, welche parallel zur Höhe des Zylinders verläuft.

Proximal und distal werden im Rahmen dieser Anmeldung in Bezug auf den Behandler verstanden. Das proximale Ende des Implantats ist dem Behandler bei der Implantation am nächsten, das distale Ende ist bei der Einführung des Implantats am Weitesten vom Behandler entfernt.

Vorzugsweise sollten die zwei Stellen, an denen das erste bzw. das zweite Beförderungselement angeordnet ist, einen relativ großen Abstand zueinander aufweisen und möglichst unmittelbar miteinander zu verbinden sein, wobei Letzteres in dem Sinne zu verstehen ist, dass ein auf dem Außengehäuse entlang führender Weg von der ersten zur zweiten Stelle möglichst direkt und geradlinig verlaufen soll. Auf diese Weise wird erreicht, dass eine Führungsvorrichtung für die Implantation, wie z. B. ein Führungsdraht oder dergleichen, auf einfache und unmittelbare Weise vom ersten zum zweiten Beförderungselement geführt werden und mit den Beförderungselementen gekoppelt werden kann, und wobei sich das Implantatgehäuse in seiner Geometrie an den Führungsdraht möglichst anpasst bzw. anschmiegt.

In einer Ausgestaltung der vorliegenden Erfindung sind besagte erste und zweite Rotationsachse zueinander parallel orientiert. Des Weiteren können die erste und zweite Rotationsachse derart ausgelegt sein, dass sie zu einer Längsachse des Implantatgehäuses senkrecht orientiert sind. Alternativ oder zusätzlich können besagte Rotationsachsen zu der Ebene, in welcher das erste bzw. das zweite Beförderungselement angeordnet ist, senkrecht orientiert sein.

Werden das erste und das zweite Beförderungselement mit einer Führungsvorrichtung für die Implantation, wie z. B. einem Führungsdraht oder Ähnlichem, gekoppelt, so bewirken die drehbar gelagerten Beförderungselemente eine Beweglichkeit des Implantats relativ zur Führungsvorrichtung. Dies ist vorteilhaft bei der Implantation, da das Implantat nicht starr mit der Führungsvorrichtung gekoppelt ist und so leichter durch Blutgefäße mit geringem Durchmesser und kleinem Kurvenradius befördert werden kann.

Bei besagter Ausrichtung der Rotationsachsen kann erreicht werden, dass sich das Implantat in seiner Länge im Wesentlichen tangential zur Biegekurve jenes Abschnitts der Führungsvorrichtung ausrichtet, an welchem sich das medizinische Implantat befindet.

Unter der Längsachse wird im Rahmen dieser Anmeldung die Achse verstanden, entlang der das Implantat seine größte Ausdehnung hat.

Gemäß einer erfindungsgemäßen Ausführungsform sind das erste und das zweite Beförderungselement dazu ausgelegt, zusammen mit einem Führungsdraht zu wirken.

Im Sinne der Erfindung sind in diesem Zusammenhang auch neben einem Führungsdraht andere Vorrichtungen mit eingeschlossen, die zur intravaskulären Beförderung von Implantaten genutzt werden können, wie beispielsweise Kathetersysteme oder dergleichen. Erfindungsgemäß ist unter dem Zusammenwirken der Beförderungselemente mit dem Führungsdraht jegliche Form einer Kopplung zu verstehen, die es ermöglicht, das medizinische Implantat über mittels des Führungsdrahts zu befördern.

In einer Ausführungsform der Erfindung weisen die Beförderungselemente jeweils ein Fassungsglied auf, wobei das Fassungsglied dazu ausgestaltet ist, einen Führungsdraht aufzunehmen. Dabei weist in bevorzugten Ausführungsformen der Erfindung das Fassungsglied die Form eines Rings, einer Öse, einer Schlaufe oder eines Hakens auf.

So kann der Führungsdraht beispielsweise durch ring- oder ösenförmige Fassungsglieder gefädelt werden. Bei Schlaufen bzw. Haken wird der Führungsdraht eingespannt bzw. eingehakt, wobei ein Mechanismus zum Einsatz kommen kann, der die Kopplung sichert, wie beispielsweise durch Einrasten oder Einklinken. Dabei sollten die Fassungsglieder entlang des Führungsdrahts beweglich sein, d. h. die Fassungsglieder sollten nicht in starrer Verbindung mit dem Führungsdraht stehen. Andere Ausführungsformen für die Fassungsglieder sind ebenfalls denkbar, die ebenfalls eine beschriebene Art der Kopplung zwischen Beförderungselemente und dem Führungsdraht bieten, wie zum Beispiel Schienen-, Klammer-, Tunnelsysteme oder Ähnliche.

In einer bevorzugten Ausführungsform der Erfindung weist das medizinische Implantat eine Fixierungsstruktur mit mindestens zwei Fassungseinheiten auf, wobei die Fixierungsstruktur einen expandierten und einen kollabierten Zustand einnehmen kann. Dabei ist die Fixierungsstruktur im expandierten Zustand dazu ausgelegt, das medizinische Implantat an einem Implantationsort zu fixieren.

In einer Ausführungsform der Erfindung weist die Fassungseinheit die Form eines Rings, einer Öse, einer Schlaufe oder eines Hakens auf.

Zum Beispiel kann die Fixierungsstruktur in Form von Beinchen, Klappen, Gerüst, Gitter, Netz oder Stent ausgeführt sein.

Im Sinne der Erfindung ist unter einem kollabierten Zustand der Fixierungsstruktur eine Form zu verstehen, in der die Fixierungsstruktur eine für die intravaskuläre Beförderung geeignete Außengeometrie aufweist. Unter einem expandierten Zustand ist eine Form zu verstehen, in der die Fixierungsstruktur eine Außengeometrie aufweist, die sich zur Fixierung/Verankerung/Befestigung der Fixierungsstruktur selbst sowie des medizinischen Implantats in einem Blutgefäß eignet.

Ist die Fixierungsstruktur ausgeführt als Beinchen, Klappen oder Gerüst, so befinden sich diese im kollabierten Zustand, wenn sie geschlossen sind, und im expandierten Zustand, wenn sie auseinandergeklappt sind. Entsprechend gilt dies für eine gitter-, netz- oder stentförmige Fixierungsstruktur, die zusammengefaltet bzw. auseinandergefaltet den kollabierten bzw. expandierten Zustand darstellen kann.

Die Fixierungsstruktur besteht vorzugsweise aus biokompatiblem Material, welches für eine dauerhafte Implantation im Blutfluss geeignet ist. Nach Erreichen des Implantationsortes kann die Fixierungsstruktur expandiert werden. Die Fassungseinheiten sind an die Fixierungsstruktur gekoppelt und sind dazu ausgestaltet, die Fixierungsstruktur im kollabierten Zustand zu halten.

Die Fassungseinheiten können mit der Fixierungsstruktur fest verbunden sein. Beispielsweise sind sie an einem proximalen und/oder distalen Ende der Fixierungsstruktur angeordnet und weisen bevorzugt die Form eines Rings oder ähnlich eines Rings auf. Ebenfalls denkbar wären Schienen-, Klammer-, Tunnelsysteme oder Ähnliche.

In einer Ausführungsform können die Fassungseinheiten mit einer Leine oder dergleichen verbunden sein. Dies würde eine Explantation des Implantats vereinfachen, indem beispielsweise die Leine über einen Katheter eingefangen wird.

Gemäß einer Ausführungsform der Erfindung sind die Fassungseinheiten der Fixierungsstruktur dazu ausgelegt, mit einem Führungsdraht zusammen zu wirken, so dass sich die Fixierungsstruktur im kollabierten Zustand befindet.

Vorzugsweise sind die Fassungseinheiten ausgestaltet, mit dem Führungsdraht derart koppelbar zu sein, sodass die Fixierungsstruktur im kollabierten Zustand gehalten wird. Dafür kann der Führungsdraht beispielsweise von den Fassungseinheiten aufgenommen werden.

So kann auch der Führungsdraht beispielsweise durch ring- oder ösenförmige Fassungseinheiten gefädelt werden, analog zu den Fassungsgliedern. Bei Schlaufen- bzw. hakenförmigen Fassungseinheiten wird der Führungsdraht eingespannt bzw. eingehakt, wobei ein Mechanismus zum Einsatz kommen kann, der die Kopplung sichert, wie beispielsweise durch Einrasten oder Einklinken. Dabei sollten die Fassungseinheiten entlang des Führungsdrahts beweglich sein, d. h. die Fassungseinheiten sollten nicht in starrer Verbindung mit dem Führungsdraht stehen. Andere Ausführungsformen für die Fassungseinheiten sind ebenfalls denkbar, die ebenfalls eine beschriebene Art der Kopplung zwischen Beförderungselemente und dem Führungsdraht bieten, wie zum Beispiel Schienen-, Klammer-, Tunnelsysteme oder ähnliche.

In einer Ausführungsform der Erfindung umfasst die Fixierungsstruktur mindestens zwei aus flexiblem Material bestehende Schenkel. Dabei sind die Schenkel im kollabierten Zustand geschlossen und im expandierten Zustand auseinandergeklappt. Weiterhin ist es denkbar, dass jeder Schenkel einen mäanderförmig und/oder zickzackförmig auslaufenden Abschnitt aufweist.

Die Schenkel sind ausgelegt, im kollabierten Zustand eine Verlängerung des Implantatgehäuses darzustellen, sodass der Gesamtdurchmesser des medizinischen Implantats nicht oder nur geringfügig vergrößert wird. An einem gewünschten Implantationsort innerhalb eines Blutgefäßes können die Schenkel in den expandierten Zustand gebracht werden. Dann ist der Gesamtdurchmesser des Implantats deutlich vergrößert, die Schenkel sind auseinandergeklappt und kontaktieren jeweils mit einem Ende die Gefäßinnenwand, sodass sie Kraft auf die Gefäßinnenwand übertragen können (z. B. durch Einklemmen oder Druck), um das medizinische Implantat im Blutgefäß zu stützen und an jener Stelle zu halten. Beispielsweise könnten die aufgeklappten Schenkel eine V-Form bilden und in der Form im Blutgefäß das Implantat fixieren. Eine V-Form zeichnet sich dadurch aus, dass sie einen anisotropen Strömungswiderstand aufweisen, d. h. ein im Blutgefäß fixiertes Implantat bietet gegenüber dem Blutstrom einen Widerstand, der von der Blutflussrichtung abhängig ist. Dadurch kann durch entsprechende Platzierung des Implantats verhindert werden, dass das Implantat nach gewisser Zeit 'wandert', d. h. mit dem Blutstrom in eine Richtung bewegt wird. In diesem Zusammenhang sind auch andere, zum genannten Zweck geeignete Formen für die Fixierungsstruktur mit anisotropem Strömungswiderstand im expandierten Zustand denkbar.

Gemäß einer Ausführung der Erfindung besteht die Fixierungsstruktur mindestens teilweise aus flexiblem Material, da sich das Implantat während der Implantation widerstandsärmer durch Blutgefäße befördern lässt und nach Fixierung des Implantats im Blutgefäß eine gewisse Anpassung der Schenkel an die zyklischen Kräfte stattfindet, die durch den Blutfluss im Gefäß herrschen, sodass die Lebensdauer des Materials verlängert wird. Dabei sollte der Grad der Flexibilität entsprechend den beschriebenen zu erfüllenden Eigenschaften gewählt werden.

Gemäß einer Ausführungsform weisen die proximalen Enden der Schenkel einen mäanderförmig auslaufenden Abschnitt auf, welcher vorteilhafterweise der Abschnitt ist, der die Gefäßinnenwand kontaktiert. Somit wird die Kontaktfläche der Fixierungsstruktur auf der Gefäßinnenwand vergrößert und dadurch der Druck, welcher von der Fixierungsstruktur auf die Gefäßinnenwand gebracht wird, reduziert. Des Weiteren wird die Elastizität der Fixierungsstruktur im Bereich der Auflageflächen erhöht. So wird erreicht, dass die Kontaktflächen tangential zur Gefäßwand verlaufen. Vorteilhaft ist dies, da einerseits der Halt des Implantats im Gefäß vergrößert wird und andererseits das Risiko eines Einschneidens in die Gefäßwand durch die Schenkel sinkt. Mit umfasst sind auch andere Formen und Muster für den Abschnitt der Schenkel, die besagten Effekt hervorrufen, wie zum Beispiel Zickzackformen, Treppenformen oder andere repetitive Geometrien.

Gemäß einer Ausführungsform der vorliegenden Erfindung ist die Fixierungsstruktur mit mindestens einer mechanischen Feder gekoppelt. Dabei ist die mechanische Feder gespannt, wenn die Fixierungsstruktur sich im kollabierten Zustand befindet, und befindet sich in der Ruhelage, wenn die Fixierungsstruktur sich im expandierten Zustand befindet.

Die erfindungsgemäße mechanische Feder kann beispielsweise in Form einer Schenkelfeder, Laschenfeder, Helixfeder oder einer ähnlichen Feder realisiert werden. Die Feder kann beispielsweise an oder integriert in oder innerhalb des Implantatgehäuses angeordnet sein. Weist die Fixierungsstruktur Beinchen oder Schenkel wie in den beschriebenen Ausführungsformen auf, so können die Beinchen oder Schenkel an ihrem dem Implantatgehäuse zugewandten Ende mit der Feder gekoppelt sein oder in eine Feder übergehen. Bei beschriebener Ausführungsform mit zwei Schenkeln, die in V-Form ausklappen, kann die Feder zwischen den zwei Schenkeln angeordnet sein (d. h. an der Spitze des Vs), wobei die Feder an einem Ende des Implantatgehäuses befestigt ist. In einer Ausführungsform verfügen die Schenkel jeweils über eine Fassungseinheit am proximalen Ende, mittels derer sie einen Führungsdraht aufnehmen können. Zu diesem Zweck müssen die Schenkel geschlossen werden, sodass die Fassungseinheiten in einer Linie ausgerichtet sind und der Führungsdraht aufgenommen werden kann. Zum Schließen der Schenkel wird dabei die mechanische Feder gespannt, die Fixierungsstruktur befindet sich im kollabierten Zustand. Wird der Führungsdraht zurückgezogen, sodass er nicht mehr von den Fassungseinheiten aufgenommen ist, nimmt die Fixierungsstruktur sofort den expandierten Zustand ein, beispielsweise durch Aufklappen der zwei Schenkel in die V-Form, hervorgerufen durch die Rückstellkraft der Feder, die sie wieder in die Ruhelage versetzt.

Gemäß einer Ausführungsform der Erfindung besteht die Fixierungsstruktur zumindest teilweise aus einem Formgedächtnismaterial, insbesondere einer Formgedächtnislegierung wie Nitinol.

Formgedächtnismaterialien zeichnen sich dadurch aus, dass sie unter bestimmten äußeren Umständen verformbar sind, aber nach einer Verformung durch einen Stimulus wieder in ihre Ausgangsform wiedererlangen können. Beispielsweise kann eine Temperaturveränderung einen solchen Stimulus darstellen.

Das Formgedächtnismaterial kann beispielsweise derart gewählt werden, dass es bei Körpertemperatur seine Ausgangsform wieder einnimmt ('Austenit-Zustand'). Vorzugsweise wird der expandierte Zustand als Ausgangsform für die Fixierungsstruktur gewählt. Die Fixierungsstruktur wird vor Implantation zunächst in den verformbaren Zustand versetzt ('Martensit-Zustand', beispielsweise durch Abkühlen), in den kollabierten Zustand geformt und mit einem Führungsdraht gekoppelt, sodass die Fassungseinheiten den Führungsdraht aufnehmen. Bei der Implantation wird das Formgedächtnismaterial durch die Körpertemperatur wieder auf eine Temperatur oberhalb der Übergangstemperatur erwärmt. Dabei wird die Fixierungsstruktur durch den Führungsdraht noch in ihrem kollabierten Zustand gehalten, dieser wird als spannungsinduzierter Martensitzustand bezeichnet. Sobald das Implantat am gewünschten Implantationsort befördert wurde und der Führungsdraht entfernt wird, erlangt die Führungsstruktur ihre Ausgangsform (den expandierten Zustand, bzw. Austenitzustand) wieder.

Die Verwendung eines Formgedächtnismaterials für den Fixiermechanismus ermöglicht seine Änderung vom kollabierten in den expandierten Zustand ohne Einsatz eines zusätzlichen mechanischen Energiespeichers.

In einer bevorzugten Ausführungsform der vorliegenden Erfindung weist das medizinische Implantat eine Greifeinheit auf. Die Greifeinheit kann beispielsweise als Ring, Öse, Haken oder dergleichen ausgestaltet und am proximalen Ende des medizinischen Implantats angeordnet sein. Dabei dient die Greifeinheit zur Anbindung des Implantats mit einer Komponente zum Schieben bzw. Ziehen des Implantats entlang des Führungsdrahts. Auch kann die Greifeinheit nach der Implantation zur Justierung des Implantationsorts bzw. zur Explantation dienen, in dem sie mittels eines Katheters oder dergleichen gegriffen und gehalten wird, sodass das medizinische Implantat bewegt werden kann.

Die vorliegende Erfindung umfasst des Weiteren ein System zur intravaskulären Implantation eines zuvor beschriebenen medizinischen Implantats, wobei das System mindestens einen flexiblen Führungsdraht und eine längliche Schiebeeinheit aufweist. Dabei sind das Implantat, der Führungsdraht und die Schiebeeinheit bevorzugt derart angeordnet sind, dass
- der Führungsdraht mit dem ersten und zweiten Beförderungselement des Implantats zusammen wirkt, wobei bevorzugt das erste und zweite Beförderungselement jeweils ein Fassungsglied aufweisen, welche den Führungsdraht aufnehmen, und dass
- die Schiebeeinheit mit dem Führungsdraht zusammenwirkt, sodass die Schiebeeinheit entlang des Führungsdrahts bewegbar ist, wobei die Schiebeeinheit dazu ausgelegt ist, das medizinische Implantat entlang des Führungsdrahts zu schieben und/oder zu ziehen.

In einer bevorzugten Ausführungsform des erfindungsgemäßen Systems zur intravaskulären Implantation umfasst besagte Schiebeeinheit einen flexiblen Schaft, im Folgenden Schieber-Schaft genannt und ein Schiebemodul, wobei der Schieber-Schaft ein Lumen umschließt und das Schiebemodul am proximalen Ende des Schieber-Schafts angeordnet ist. Dabei ist der Führungsdraht bevorzugt zumindest zum Teil innerhalb des Lumens angeordnet und das Schiebemodul wirkt derart mit dem medizinischen Implantat zusammen, dass das Implantat durch das Schiebemodul zumindest gehalten, gezogen und/oder geschoben werden kann. Auf diese Weise kann durch die Schiebeeinheit die Position des Implantats im Bezug zum Führungsdraht durch das Schiebemodul kontrolliert werden.

Bevorzugt nehmen die Fassungsglieder den Führungsdraht so auf, dass das Implantat an den Führungsdraht gekoppelt ist, sich aber frei entlang desselben bewegen lässt. Umsetzbar ist eine solche Kopplung beispielsweise, in dem die Fassungsglieder als ring-, ösen-, Schlaufen-, haken-, schienen-, klammer-, tunnelförmig gestaltet sind oder in einer den beschriebenen ähnlichen Form ausgestaltet sind.

Durch das erfindungsgemäße System und beschriebene Ausführungsformen wird ein System zur intravaskulären Implantation geschaffen, welches auf einfache Weise ermöglicht, Implantate durch Blutgefäße mit geringem Durchmesser und kleinem Kurvenradius zu befördern. Darüber hinaus weist das System einen unkomplizierten Aufbau auf und kann herstellerseitig vormontiert werden, wodurch eine mühsame und zeitaufwändige Montage unmittelbar vor Ort der Implantation wegfällt.

In einer Ausführungsform des erfindungsgemäßen Systems sind das medizinische Implantat und der Führungsdraht derart angeordnet, dass die Fixierungsstruktur an proximaler Position und das Implantatgehäuse an distaler Position angeordnet ist. Alternativ kann das Implantatgehäuse an proximaler Position und die Fixierungsstruktur an distaler Position angeordnet sein.

Die Schiebeeinheit kann als Pusher-Katheter ausgestaltet sein, der z. B. typischerweise für die Katheterisierung des Herzkreislaufsystems eingesetzt wird. Es kann auch ein System als Schiebeeinheit gewählt werden, welche das medizinische Implantat über besagte Greifeinheit greifen und halten kann, um es schieben oder ziehen zu können. Auf diese Weise sind bei der Implantation dem Behandler mehr Freiheiten für die Beförderung und Platzierung des Implantats gegeben, vor allem wird somit die Justierung der Implantatposition vereinfacht. Ist das Implantat so am Führungsdraht angeordnet, dass sich die Fixierungsstruktur an proximaler und das Implantatgehäuse an distaler Position befindet, so können die Fixierungsstruktur selbst, Teile derselben oder die Fassungseinheiten als Greifeinheiten dienen. Ist hingegen das Implantatgehäuse an proximaler und die Fixierungsstruktur an distaler Position angeordnet, so kann z. B. besagte Greifeinheit am proximalen Ende des Implantatgehäuses angeordnet sein, die von der Schiebeeinheit gegriffen und gehalten werden kann. Eine solche Schiebeeinheit mit Greiffunktion kann derart in Zusammenwirkung mit dem Führungsdraht gestaltet sein, sodass der Griff gelöst wird, wenn der Führungsdraht zurückgezogen wird.

In einer Ausführungsform des beschriebenen Systems zur intravaskulären Implantation eines beschriebenen medizinischen Implantats umfasst das System keine Schiebeeinheit. Stattdessen weist das System einen langen flexiblen Schaft, welcher ein Lumen umschließt und ein proximales und ein distales Ende besitzt. Das Implantat und der Führungsdraht sind mindestens teilweise innerhalb des Lumens angeordnet, wobei der Führungsdraht von den Beförderungselementen aufgenommen ist. Vorzugsweise ist das Implantat dabei nahe dem distalen Ende des flexiblen Schafts angeordnet, wobei die Fixierungsstruktur des Implantats zum distalen Ende des flexiblen Schafts orientiert ist, sprich in Vorschubrichtung des Schafts im Gefäßsystem. Die Fixierungsstruktur wird dabei durch den Führungsdraht im kollabierten Zustand gehalten. Der flexible Schaft wird, zusammen mit Führungsdraht und Implantat im inneren Lumen, intravaskulär zum Implantationsort vorgeschoben, wo das Implantat aus dem flexiblen Schaft mithilfe des folgenden Vorgangs freigesetzt wird. Mit dem Führungsdraht wird das Implantat innerhalb des flexiblen Schafts so weit in distaler Richtung vorgeschoben, bis die Fixierungsstruktur teilweise aus dem flexiblen Schaft herausragt. Das Implantatgehäuse sollte sich dabei noch innerhalb des flexiblen Schafts befinden. Anschließend wird der Führungsdraht in proximaler Richtung zurückgezogen, sodass die Fixierungsstruktur in den expandierten Zustand gebracht wird, sodass der distale Teil der Fixierungsstruktur an der Gefäßinnenwand abstützt ist, wobei der proximale Teil der Fixierungsstruktur innerhalb des flexiblen Schafts angeordnet ist. Anschließend wird der flexible Schaft in proximaler Richtung zurückgezogen, wobei sich das Implantat aus dem flexiblen Schaft gelöst und durch die Fixierungsstruktur am Implantationsort gehalten. Auf diese Weise wird ein ruckartiges Lösen des Implantats verhindert und ein kontrolliertes Freisetzen aus dem flexiblen Schaft ermöglicht.

Gemäß einer erfindungsgemäßen Ausführungsform des Systems zur intravaskulären Implantation weist das medizinische Implantat eine zuvor beschriebene Fixierungsstruktur mit mindestens zwei Fassungseinheiten wie zuvor beschrieben auf, wobei die Fixierungsstruktur einen expandierten und einen kollabierten Zustand einnehmen kann. Des Weiteren sind die Fassungseinheiten dazu ausgelegt, mit dem Führungsdraht zusammen zu wirken, in dem der Führungsdraht durch die Fassungseinheiten aufgenommen wird, sodass sich die Fixierungsstruktur im kollabierten Zustand befindet. Dabei ist die Fixierungsstruktur dazu ausgelegt, in den expandierten Zustand überzugehen, sobald der Führungsdraht nicht mit den Fassungseinheiten zusammenwirkt.

Gemäß einer Ausführungsform sind die Fassungseinheiten in Form von Ringen, Ösen, Schlaufen, Haken, Schienen, Klammern, Tunneln oder in einer den beschriebenen ähnlichen Form ausgestaltet. Ähnlich wie für die Fassungsglieder sollten auch die Fassungseinheiten bevorzugt so gestaltet sein, dass sie eine Kopplung der Fixierungsstruktur an den Führungsdraht gewährleisten, wobei es jedoch möglich sein sollte, dass die Fixierungsstruktur und mit ihr das medizinische Implantat entlang des Führungsdrahts bewegbar sind. Vorzugsweise wird die Fixierungsstruktur im kollabierten Zustand gehalten, wenn die Fassungseinheiten mit dem Führungsdraht gekoppelt sind. Sobald diese Kopplung entfällt, beispielsweise in dem der Führungsdraht zurückgezogen wird, nimmt die Fixierungsstruktur den expandierten Zustand ein.

Die Erfindung ist nachfolgend beispielhaft, anhand von einem in Zeichnungen dargestellten Ausführungsbeispiel, näher erläutert. Es zeigen in schematischer Darstellung:
- Fig. 1: eine erste erfindungsgemäße Ausführungsform des medizinischen Implantats in Schrägansicht und Seitenansicht;
- Fig. 2: beispielhafte Ausführungsformen der Fixierungsstruktur gemäß Ausführungsformen der vorliegenden Erfindung;
- Fig. 3: weitere beispielhafte Ausführungsformen der Fixierungsstruktur gemäß Ausführungsformen der vorliegenden Erfindung;
- Fig. 4: beispielhafte Ausführungsformen der Beförderungselemente und Fassungsglieder gemäß Ausführungsformen der vorliegenden Erfindung;
- Fig. 5: einen beispielhaften Vorgang für den Wechsel zwischen kollabiertem und expandiertem Zustand für eine Ausführungsform des erfindungsgemäßen medizinischen Implantats;
- Fig. 6: einen weiteren beispielhaften Implantationsvorgang für eine Ausführungsform des erfindungsgemäßen medizinischen Implantats;
- Fig. 7: Ausführungsformen für das erfindungsgemäße System zur intravaskulären Implantation eines medizinischen Implantats;
- Fig. 8: Ausführungsform der Schiebeeinheit des erfindungsgemäßen Systems zur intravaskulären Implantation eines medizinischen Implantats;
- Fig. 9: Ausführungsform für das erfindungsgemäße System zur intravaskulären Implantation eines medizinischen Implantats innerhalb einer Biegung mit kleinem Kurvenradius;
- Fig. 10: Detailansicht einer Ausführungsform der Fixierungsstruktur gemäß Ausführungsformen der vorliegenden Erfindung für das erfindungsgemäße System zur intravaskulären Implantation eines medizinischen Implantats; und
- Fig. 11: eine alternative Ausführungsform eines medizinischen Implantats sowie Systems zur intravaskulären Implantation eines medizinischen Implantats.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Auch sind die in den Figuren dargestellten Ausführungsformen und/oder Merkmale des erfindungsgemäßen medizinischen Implantats sowie des Systems zur intravaskulären Implantation eines solchen Implantats untereinander kombinierbar und sollen nicht auf das in der jeweiligen Figur gezeigte Beispiel und seinen Merkmalen beschränkt sein.

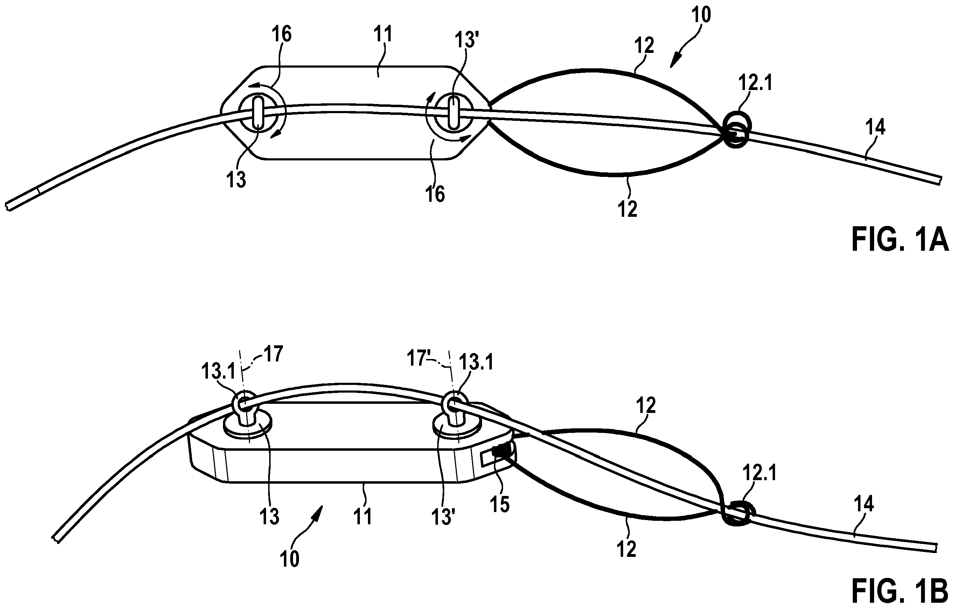

Fig. 1 zeigt eine erste erfindungsgemäße Ausführungsform des medizinischen Implantats in Seitenansicht 1a und Schrägansicht 1b. Dabei weist das medizinische Implantat 10 ein Implantatgehäuse 11 und eine Ausführungsform der Fixierungsstruktur 12 mit zwei Schenkeln auf, an deren distalen Enden Fassungseinheiten 12.1 angeordnet sind. Die proximalen Enden der zwei Schenkel der Fixierungsstruktur 12 sind mit einer mechanischen Feder 15 gekoppelt, die am Implantatgehäuse 11 angeordnet ist. Zwei Beförderungselemente 13, 13' mit zwei Fassungsgliedern in Form von Ösen 13.1 sind auf einer Seitenfläche des Implantatgehäuses angeordnet. Die zwei Beförderungselemente 13, 13' sind dabei drehbar in Drehrichtung 16, um Rotationsachse 17, 17' gelagert. Führungsdraht 14 ist durch Fassungsglieder 13.1 und Fassungseinheiten 12.1 aufgenommen, sodass die Fixierungsstruktur im kollabierten Zustand gehalten wird und sodass sich das medizinische Implantat 10 entlang des Führungsdrahts 14 bewegen lässt.

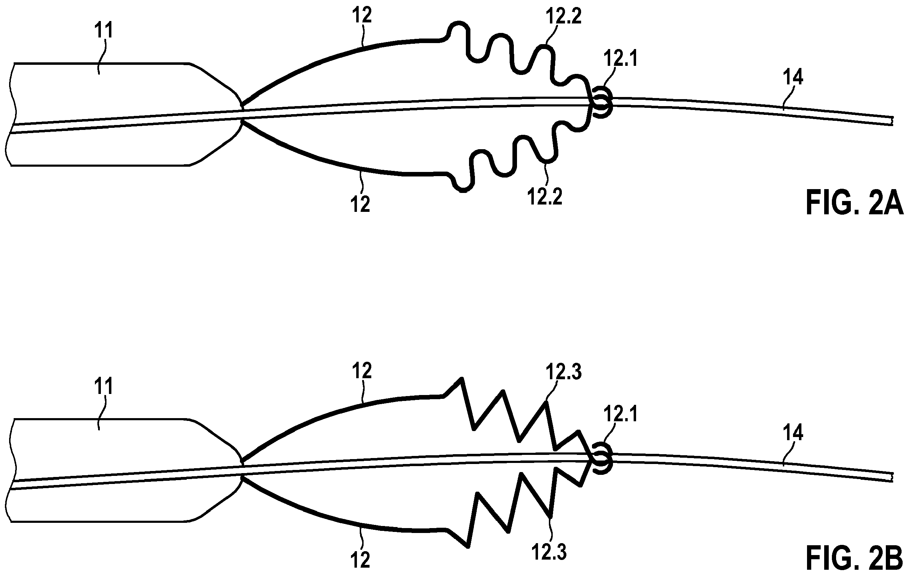

Fig. 2 zeigt zwei beispielhafte Ausführungsformen der Fixierungsstruktur 2a und 2b gemäß Ausführungsformen der vorliegenden Erfindung. In 2a und 2b sind jeweils das mit der Fixierungsstruktur 12 gekoppelte Ende des Implantatgehäuses 11 und einer beispielhaften Ausführungsform der Fixierungsstruktur 12 mit zwei Schenkeln dargestellt, wobei Führungsdraht 14 durch die Fassungseinheiten 12.1 an den vom Implantatgehäuse 11 wegführenden Enden der Fixierungsstruktur 12 aufgenommen ist. Dabei weisen in 2a die zwei Schenkel der Fixierungsstruktur 12 jeweils einen mäanderförmig auslaufenden Abschnitt 12.2 auf; in 2b weisen die zwei Schenkel der Fixierungsstruktur 12 jeweils einen zickzackförmig auslaufenden Abschnitt 12.3 auf.

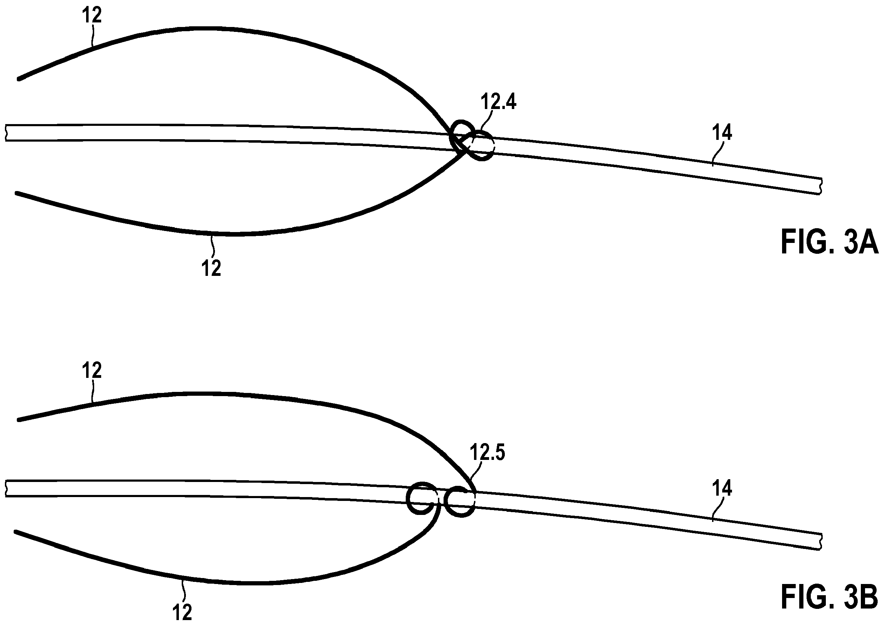

Fig. 3 zeigt weitere beispielhafte Ausführungsformen der Fixierungsstruktur 3a und 3b gemäß Ausführungsformen der vorliegenden Erfindung. In 3a und 3b ist der Führungsdraht 14 durch die Fassungseinheiten 12.4 bzw. 12.5 der Fixierungsstruktur 12 aufgenommen, sodass sich die Fixierungsstruktur 12 im kollabierten Zustand befindet. Dabei sind in 3a die Fassungseinheiten in Form von Ösen 12.4; in 3b in Form von Haken 12.5 dargestellt.

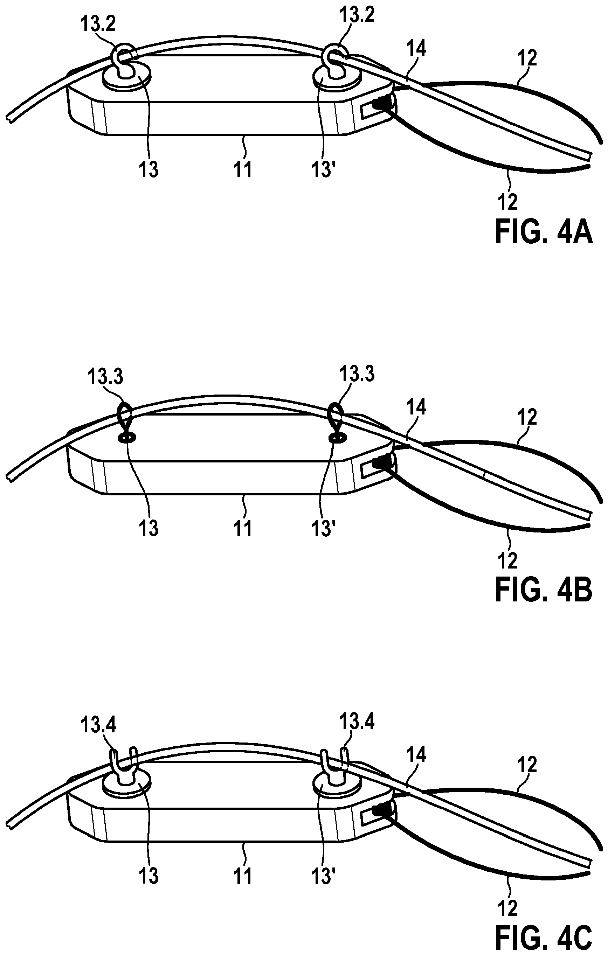

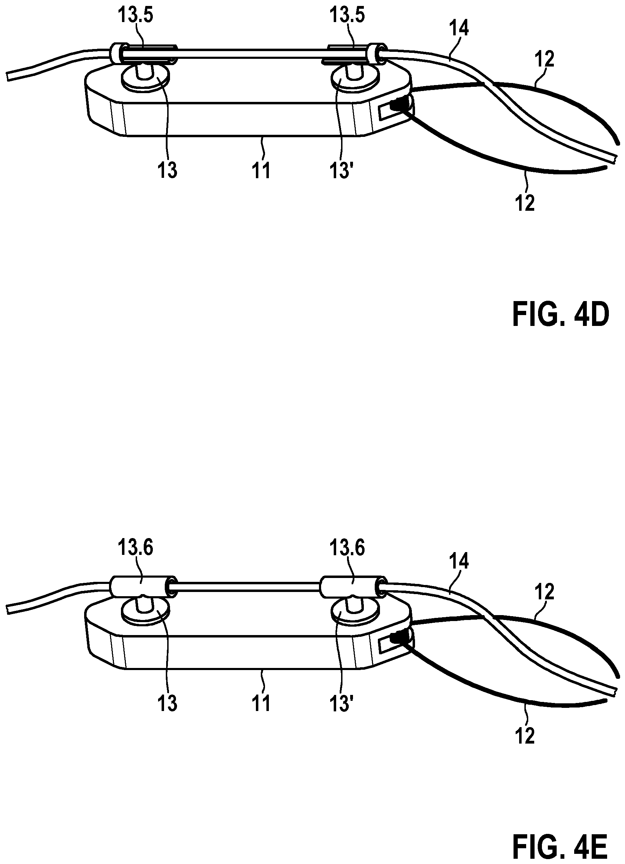

Fig. 4 zeigt beispielhafte Ausführungsformen der Beförderungselemente 13, 13' und Fassungsglieder 13.2... 13.6 gemäß Ausführungsformen der vorliegenden Erfindung. Schematisch dargestellt ist das Implantatgehäuse 11 mit jeweils zwei Beförderungselementen 13, 13' und verschiedenen Ausführungsformen der Fassungsglieder 13.2... 13.6, eine Ausführungsform der Fixierungsstruktur 12 mit zwei Schenkeln im kollabierten Zustand sowie Führungsdraht 14. Der Führungsdraht ist von den Fassungsgliedern 13.2... 13.6 aufgenommen, sodass sich das medizinische Implantat mit dem Führungsdraht gekoppelt ist, sich jedoch entlang desselben bewegbar ist. Bei den beispielhaften Ausführungsformen der Fassungsglieder handelt es sich dabei um hakenförmige 13.2, schlaufenförmige 13.3, klammerförmige 13.4, schienenförmige 13.5 und tunnelförmige 13.6 Fassungsglieder.

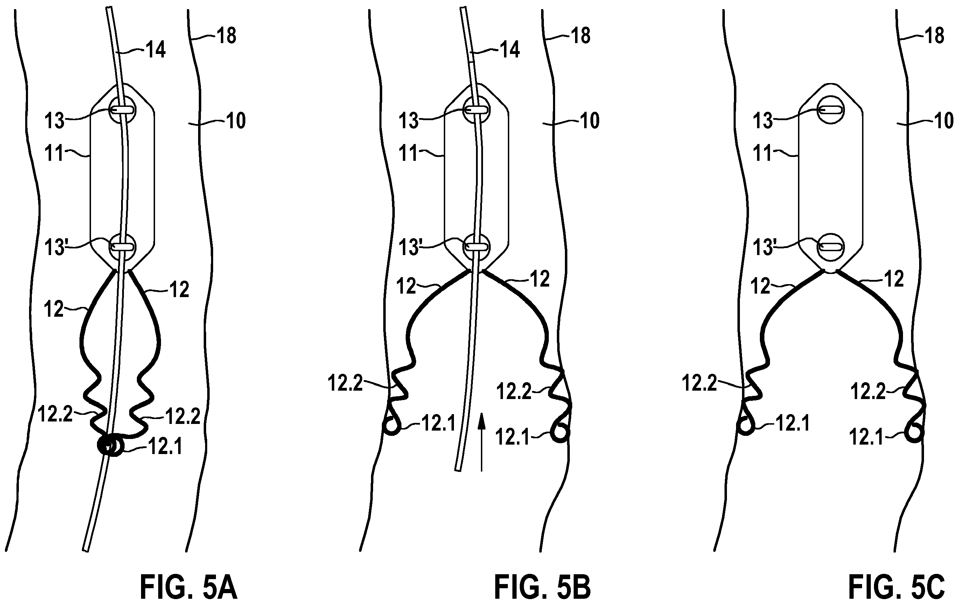

Fig. 5 zeigt einen beispielhaften Vorgang für den Wechsel zwischen kollabiertem und expandiertem Zustand für die Fixierungsstruktur anhand einer Ausführungsform des erfindungsgemäßen medizinischen Implantats. Dabei zeigen 5a, 5b und 5c das medizinische Implantat 10 mit Implantatgehäuse 11 und eine mögliche Ausführungsform der Fixierungsstruktur 12 mit zwei Schenkeln, wobei die Schenkel jeweils einen mäanderförmig auslaufenden Abschnitt 12.2 aufweisen. Das Implantat befindet sich dabei innerhalb eines Lumens 18. In Schritt 5a ist der Führungsdraht 14 durch die Fassungseinheiten 12.1 an den vom Implantatgehäuse 11 wegführenden Enden der Fixierungsstruktur 12 sowie durch die Beförderungselemente 13, 13' am Implantatgehäuse aufgenommen, sodass die Fixierungsstruktur 12 sich im kollabierten Zustand befindet und das Implantat 10 entlang des Führungsdrahts 14 beweglich ist. In Schritt 5b wird der Führungsdraht 14 aus den Fassungseinheiten 12.1 herausgezogen (entweder in Pfeilrichtung, oder in Gegenrichtung des Pfeils; in letzterem Fall würde der Führungsdraht zuerst aus den Beförderungselementen 13, 13', dann aus Fassungseinheiten 12.1 herausgezogen werden). Sobald der Führungsdraht 14 nicht mehr durch die Fassungseinheiten aufgenommen ist, nimmt die Fixierungsstruktur 12 einen expandierten Zustand ein. Dafür klappen die zwei Schenkel der Fixierungsstruktur 12 in radialer Richtung ab, wobei sich die Enden der Schenkel, beim gezeigten Beispiel an den mäanderförmig verlaufenden Abschnitten 12.2, gegen die Innenwand des Lumens 18 abstützt. Dann kann der Führungsdraht 14 entfernt werden, wie in 5c dargestellt, und das Implantat wird durch die Fixierungsstruktur 12 im Lumen 18 fixiert. Auf gezeigte Weise kann das Implantat 10 beispielsweise in einem Blutgefäß implantiert werden.

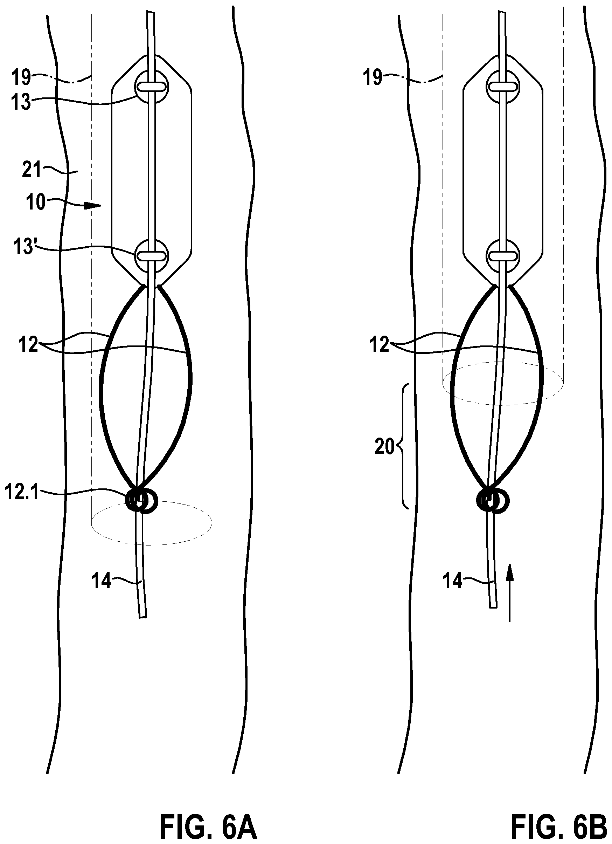

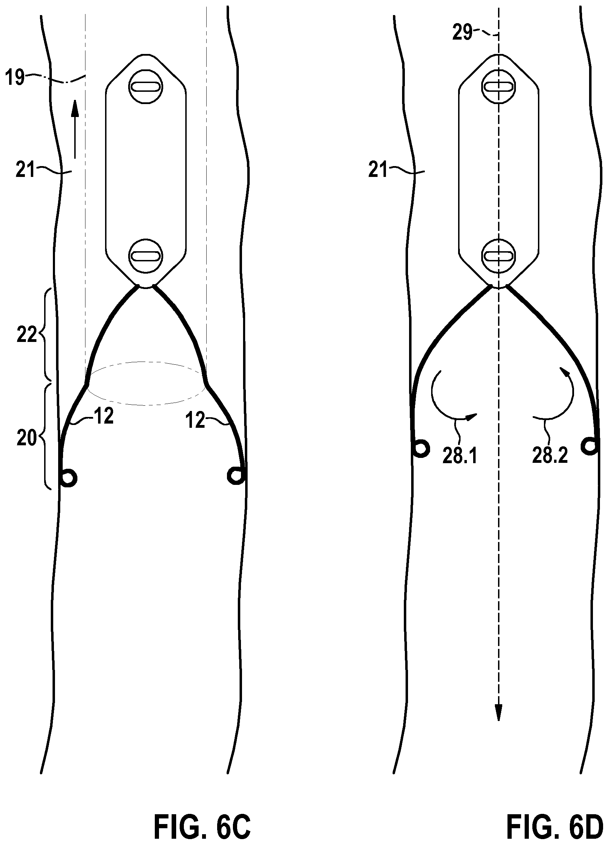

Fig. 6 zeigt einen weiteren beispielhaften Implantationsvorgang für eine Ausführungsform des erfindungsgemäßen medizinischen Implantats 10 mit den Schritten 6a...6d. Das Implantat 10 befindet sich in Schritt 6a im Innenlumen eines flexiblen Schafts 19, wobei Beförderungselemente 13, 13' sowie Fassungseinheiten 12.1 Führungsdraht 14 aufnehmen, sodass Implantat 10 entlang des Führungsdrahts 14 beweglich ist und sich Fixierungsstruktur 12 im kollabierten Zustand befindet. Der flexible Schaft ist in ein Blutgefäß 21 bis zum gewünschten Implantationsort vorgeschoben worden. In Schritt 6b wird der flexible Schaft 19 so weit in Pfeilrichtung bewegt, bis zumindest ein Abschnitt 20 der Fixierungsstruktur 12 aus dem flexiblen Schaft 19 hervorragt. In Schritt 6c wurde der Führungsdraht 14 herausgezogen, sodass Fixierungsstruktur 12 den expandierten Zustand einnimmt. Der aus dem flexiblen Schaft 19 hervorragende Abschnitt 20 der Fixierungsstruktur 12 stützt sich an der Gefäßinnenwand des Blutgefäßes 21 ab. Der sich innerhalb des flexiblen Schafts befindliche Abschnitt 22 der Fixierungsstruktur 12 stützt sich leicht an der Innenwand des flexiblen Schafts 19 ab. Anschließend wird der flexible Schaft 19 in Pfeilrichtung entfernt, sodass das Implantat 10 vollständig aus dem flexiblen Schaft freigesetzt wird und am Implantationsort fixiert, wie in 6d dargestellt. Das Entfernen, d. h. Zurückziehen des Schafts 19 sollte vorsichtig und nicht ruckartig erfolgen, da ansonsten das Implantat 10 mitgezogen werden könnte. Vorzugsweise ist die Fixierungsstruktur so ausgestaltet, dass der Winkel 28.1 und 28.2, den ein Schenkel jeweils mit der Blutflussrichtung 29 einschließt, kleiner sein sollte als 90°. Beispielsweise können die Schenkel eine V-Form bilden. Auf diese Weise erhält das Implantat 10 einen anisotropen Strömungswiderstand, d. h. ein im Blutgefäß fixiertes Implantat bietet gegenüber dem Blutstrom einen Widerstand, der von der Blutflussrichtung abhängig ist. Dadurch kann verhindert werden, dass das Implantat nach gewisser Zeit 'wandert', d. h. mit dem Blutstrom in eine Richtung bewegt wird.

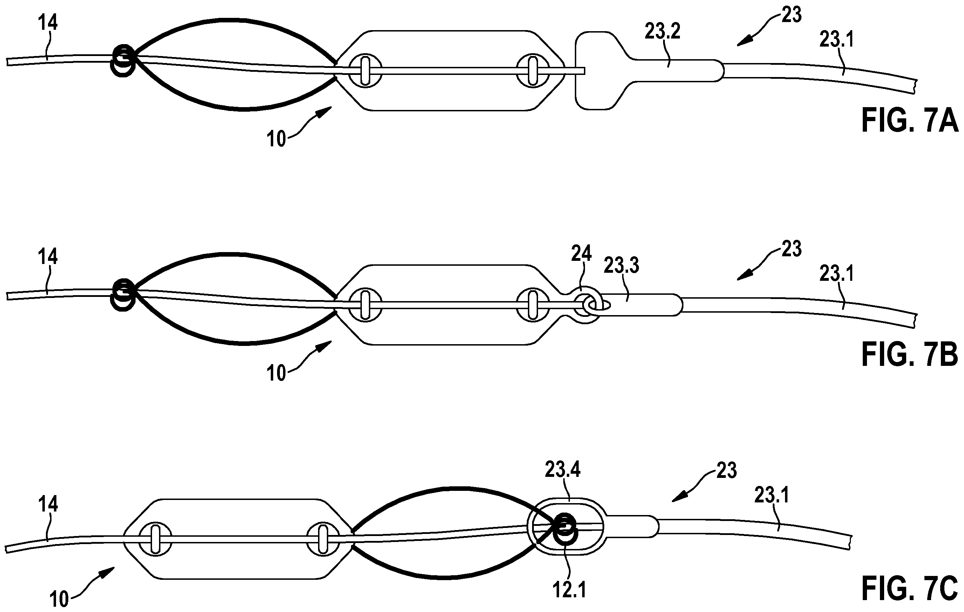

Fig. 7 zeigt Ausführungsformen für das erfindungsgemäße System zur intravaskulären Implantation eines medizinischen Implantats 10, aufweisend einen Führungsdraht 14 und eine Schiebeeinheit 23. In den Beispielen 7a, 7b und 7c wird das System in Anordnungen gezeigt, die für die Beförderung des Implantats zum Implantationsort geeignet wären. 7a, 7b und 7c zeigen das Implantat 10, das mit dem Führungsdraht so zusammenwirkt, dass es sich entlang desselben bewegen lässt und wobei Fixierungsstruktur 12 sich im kollabierten Zustand befindet. Des Weiteren ist Schiebeeinheit 23 mit Schieber-Schaft 23.1 dargestellt, wobei letzterer ein Innenlumen aufweist, in dem Führungsdraht 14 mindestens teilweise angeordnet ist. In 7a weist die Schiebeeinheit ein Schiebemodul 23.2 auf, welches dazu genutzt werden kann, um Implantat 10 vorzuschieben. Beim Ausführungsbeispiel in 7b verfügt das medizinische Implantat über eine Greifeinheit 24, während die Schiebeeinheit 23 so gestaltet ist, dass Greifeinheit 24 über Schiebemodul 23.3 gegriffen und gehalten werden kann. Auf diese Weise kann dann Implantat 10 über die Schiebeeinheit 23 geschoben oder gezogen werden. In 7c ist ein Ausführungsbeispiel gezeigt, bei dem Schiebeeinheit 23 ein Schiebemodul 23.4 aufweist, welches Fassungseinheiten 12.1 aufnimmt. Somit ist es möglich, Implantat 10 mit der Schiebeeinheit zu ziehen oder zu schieben.

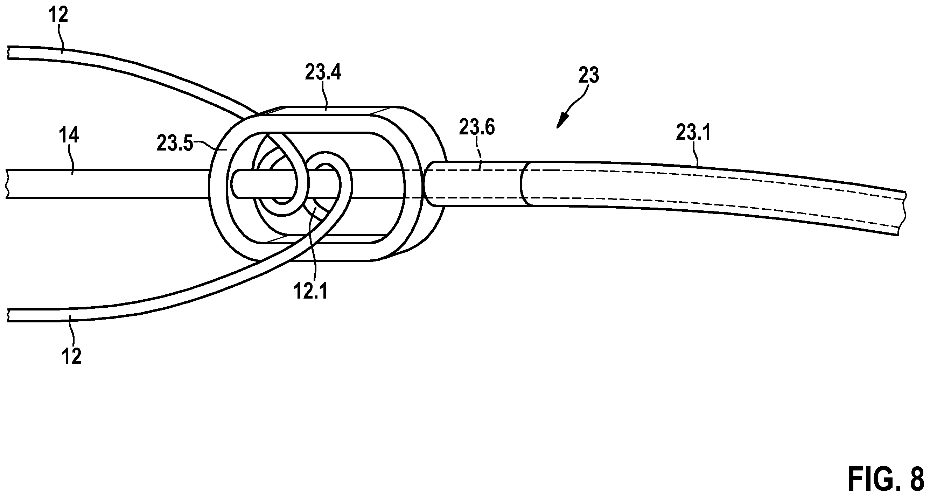

Fig. 8 zeigt eine Detaildarstellung der Schiebeeinheit aus dem Ausführungsbeispiel 7c aus Fig. 7 des erfindungsgemäßen Systems zur intravaskulären Implantation eines medizinischen Implantats 10. Die Fassungseinheiten 12.1 der zwei Schenkel der Fixierungsstruktur 12 nehmen Führungsdraht 14 auf, sodass sich Fixierungsstruktur 14 im kollabierten Zustand befindet. Gleichzeitig ist das Schiebemodul 23.4 derart gestaltet, dass es Führungsdraht 14 aufnehmen kann, in dem der Führungsdraht 14 über die Durchführungen 23.5 und 23.6 in den Schieber-Schaft 23.1 einbringbar ist. Der Aufbau ermöglicht das Ziehen oder Schieben des Implantats 10 mittels der Schiebeeinheit 23 mittels der Fassungseinheiten 12.1.

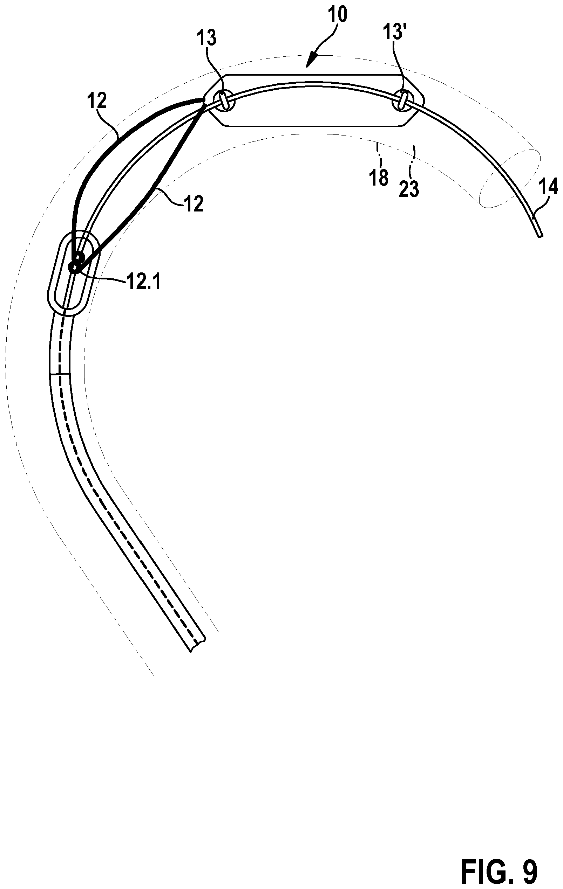

Fig. 9 zeigt eine Ausführungsform für das erfindungsgemäße System zur intravaskulären Implantation eines medizinischen Implantats 10 mit Schiebeeinheit 23 wie in 7c, Fig. 7 dargestellt, schematisch innerhalb einer Biegung mit kleinem Kurvenradius innerhalb eines Lumens 18. Führungsdraht 14 führt durch Lumen 18, wobei Führungsdraht 14 durch die Beförderungselemente 13, 13' sowie Fassungseinheiten 12.1 der Fixierungsstruktur 12 aufgenommen sind, sodass sich Implantat 10 mithilfe der Schiebeeinheit 23 entlang des Führungsdrahts 14 ziehen oder schieben lässt. In Fig. 9 ist schematisch dargestellt, dass sich aufgrund der drehbar gelagerten Beförderungselemente 13, 13' das Implantat 10 sich in seiner Länge der Biegung des Führungsdrahts 14 anpasst, sodass es sich mit weniger Widerstand durch das gebogene Lumen 18 befördern lässt.

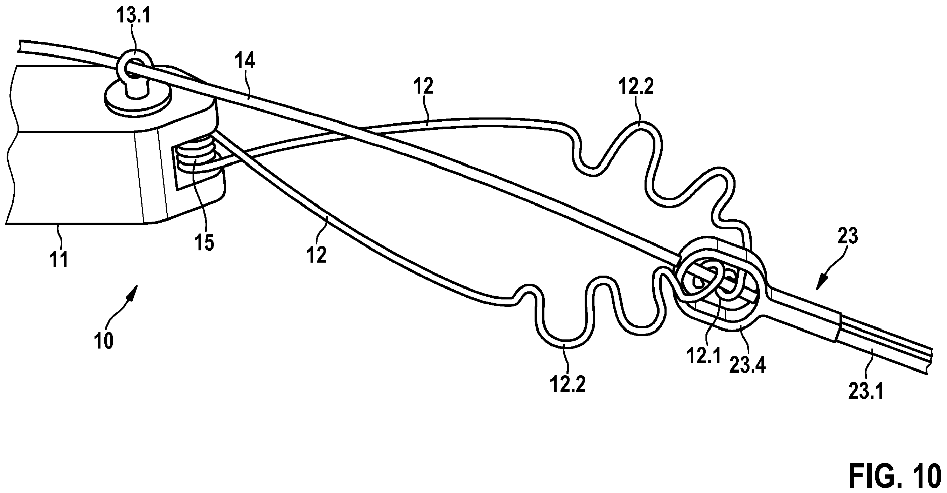

Fig. 10 zeigt eine Detailansicht einer bevorzugten Ausführungsform des medizinischen Implantats 10, insbesondere der Fixierungsstruktur 12 sowie des für das erfindungsgemäße System zur intravaskulären Implantation eines medizinischen Implantats 10. Gezeigt ist Führungsdraht 14, das der Fixierungsstruktur zugewandte Ende des Implantatgehäuses 11, Fixierungsstruktur 12 mit zwei Schenkeln, wobei die zwei Schenkel jeweils einen mäanderförmig auslaufenden Abschnitt 12.2 aufweisen und an ihrem dem Implantatgehäuse 11 zugewandten Ende mit einer mechanischen Feder 15 gekoppelt sind, sowie Schiebeeinheit 23 mit Schiebemodul 23.4 und Schieber-Schaft 23.1. Dabei ist der Führungsdraht durch die Fassungsglieder 13.1 der Beförderungselemente 13, 13' am Implantatgehäuse, durch die Fassungseinheiten 12.1 sowie durch Schiebeeinheit 23 aufgenommen, sodass sich das Implantat 10 mittels der Schiebeeinheit 23 entlang des Führungsdrahts 14 zieh- oder schiebbar gestaltet ist.

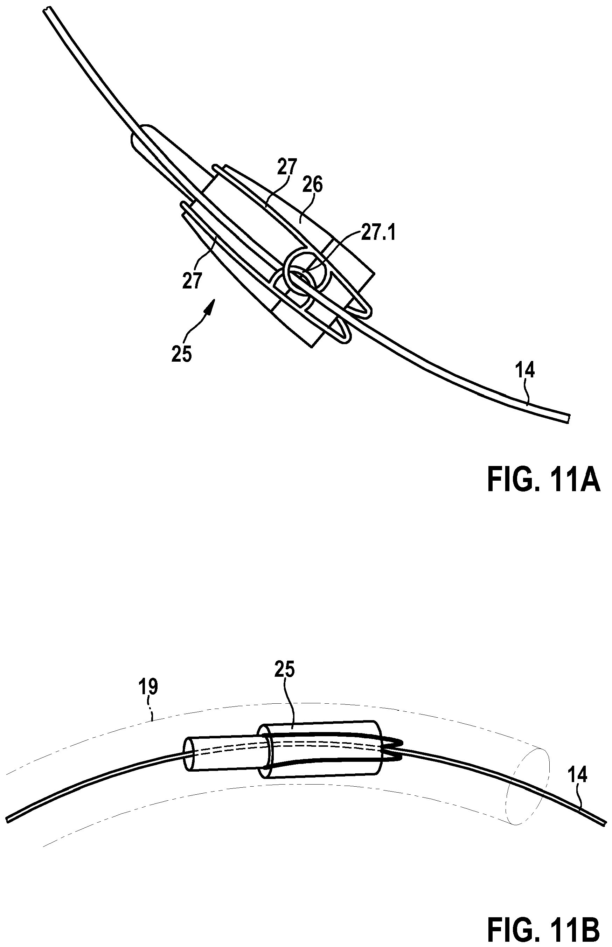

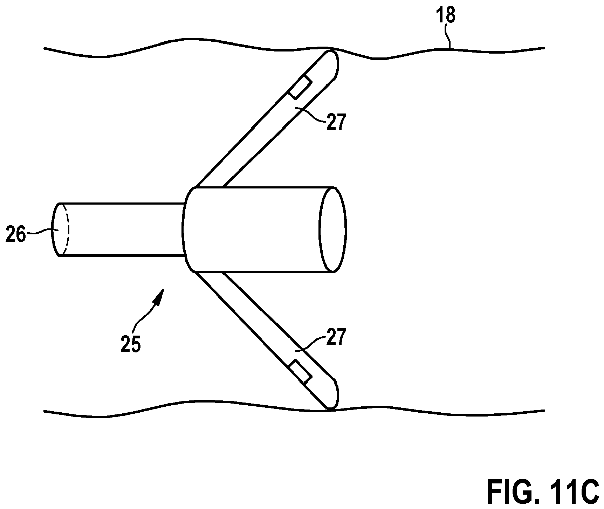

Fig. 11 zeigt eine alternative Ausführungsform des medizinischen Implantats 25 sowie des Systems zur intravaskulären Implantation eines medizinischen Implantats 25. Gezeigt werden ein Implantat 25 aufweisend ein Implantatgehäuse 26, eine Fixierungsstruktur 27 mit zwei Schenkeln in Form von Bögen sowie Fassungseinheiten 27.1, welche an den Bögen angeordnet sind. 11a zeigt die Ausführungsform des Implantats 25 mit der Fixierungsstruktur 27 im kollabierten Zustand, wobei Fassungseinheiten 27.1 einen Führungsdraht 14 aufgenommen haben, sodass das Implantat 25 entlang des Führungsdrahts beweglich ist. 11b zeigt einen möglichen Aufbau zur Implantation der Ausführungsform des Implantats 25, wobei Implantat 25 am Führungsdraht 14 innerhalb eines flexiblen Schafts 19 angeordnet ist. Mögliche Implantationsvorgänge für Implantat 25 sind analog zu jenen zuvor beschriebenen Verfahren zur Implantation gemäß der vorliegenden Erfindung. 11c zeigt die Ausführungsform des medizinischen Implantats 25, das in einem Lumen 18 (z. B. in einem Blutgefäß) fixiert ist. Dabei befindet sich Fixierungsstruktur 27 in expandierter Form, d. h. die Bögen sind aufgeklappt und stützen das Implantatgehäuse 26 innerhalb des Lumens 18 ab.

### Bezugszeichen

- 10: Medizinisches Implantat
- 11: Implantatgehäuse
- 12: Fixierungsstruktur
- 12.1, 12.4, 12.5: Fassungseinheiten
- 12.2: Mäanderförmig auslaufender Abschnitt
- 12.3: Zickzackförmig auslaufender Abschnitt
- 13, 13': Beförderungselemente
- 13.1...13.6: Fassungsglieder
- 14: Führungsdraht
- 15: Mechanische Feder
- 16: Drehrichtung der Beförderungselemente
- 17, 17': Rotationsachse der Beförderungselemente
- 18: Lumen
- 19: Flexibler Schaft
- 20: aus Schaft hervorragender Abschnitt
- 21: Blutgefäß
- 22: Innerhalb des Schafts befindlicher Abschnitt
- 23: Schiebeeinheit
- 23.1: Schieber-Schaft
- 23.2, 23.3, 23.4: Schiebemodul
- 23.5, 23.6: Durchführungen
- 24: Greifeinheit
- 25: Medizinisches Implantat
- 26: Implantatgehäuse
- 27: Fixierungsstruktur
- 27.1: Fassungseinheiten
- 28.1, 28.2: Winkel zw. Schenkel und Blutflussrichtung
- 29: Blutflussrichtung

## Patentansprüche

1. Medizinisches Implantat (10) zur intravaskulären Implantation, aufweisend ein Implantatgehäuse (11) und mindestens ein erstes und ein zweites Beförderungselement (13, 13'), wobei das erste und das zweite Beförderungselement (13, 13') in einer Ebene am Implantatgehäuse (11) angeordnet sind, wobei das erste Beförderungselement (13) drehbar um eine erste Rotationsachse (17) und das zweite Beförderungselement (13') drehbar um eine zweite Rotationsachse (17') gelagert ist.

2. Medizinisches Implantat (10) nach Anspruch 1, wobei das Implantatgehäuse (11) eine längliche Form mit einem proximalen und einem distalen Ende aufweist, wobei das erste Beförderungselement (13) nahe dem proximalen Ende und das zweite Beförderungselement (13') nahe dem distalen Ende angeordnet ist.

3. Medizinisches Implantat (10) nach Anspruch 1 oder 2, wobei das erste und das zweite Beförderungselement (13, 13') an mindestens einer seitlichen Außenfläche des Implantatgehäuses (11) angeordnet sind.

4. Medizinisches Implantat (10) nach mindestens einem der Ansprüche 1 bis 3, wobei die erste und die zweite Rotationsachse (17, 17')
- zueinander parallel orientiert sind, und/oder
- zu einer Längsachse des Implantatgehäuses (11) senkrecht orientiert sind, und/oder
- zu der Ebene, in welcher das erste und das zweite Beförderungselement (13, 13') angeordnet sind, senkrecht orientiert sind.

5. Medizinisches Implantat (10) nach einem der vorhergehenden Ansprüche, wobei das erste und das zweite Beförderungselement (13, 13') dazu ausgelegt sind, zusammen mit einem Führungsdraht (14) zu wirken.

6. Medizinisches Implantat (10) nach Anspruch 5, wobei die Beförderungselemente (13, 13') jeweils ein Fassungsglied (13.1...13.6) aufweisen, und das Fassungsglied (13.1...13.6) dazu ausgestaltet ist, einen Führungsdraht (14) aufzunehmen.

7. Medizinisches Implantat (10) nach Anspruch 6, wobei das Fassungsglied (13.1...13.6) die Form eines Rings, einer Öse, einer Schlaufe oder eines Hakens aufweist.

8. Medizinisches Implantat (10) nach mindestens einem der vorhergehenden Ansprüche, wobei das medizinische Implantat (10) eine Fixierungsstruktur (12) mit mindestens zwei Fassungseinheiten (12.1, 12.4, 12.5) aufweist, wobei die Fixierungsstruktur (12) einen expandierten und einen kollabierten Zustand einnehmen kann, und wobei die Fixierungsstruktur (12) im expandierten Zustand dazu ausgelegt ist, das medizinische Implantat (10) an einem Implantationsort zu fixieren.

9. Medizinisches Implantat (10) nach Anspruch 8, wobei die Fassungseinheiten (12.1, 12.4, 12.5) der Fixierungsstruktur (12) dazu ausgelegt sind, mit einem Führungsdraht (14) zusammen zu wirken, sodass sich die Fixierungsstruktur (12) im kollabierten Zustand befindet.

10. Medizinisches Implantat (10) nach mindestens einem der Ansprüche 8 oder 9, wobei die Fixierungsstruktur (12) mindestens zwei aus flexiblem Material bestehende Schenkel umfasst, die
- im kollabierten Zustand geschlossen sind, und
- im expandierten Zustand auseinandergeklappt sind,
und wobei jeder Schenkel einen mäanderförmig und/oder zickzackförmig auslaufenden Abschnitt (12.2, 12.3) aufweist.

11. Medizinisches Implantat (10) nach mindestens einem der Ansprüche 8 bis 10, wobei die Fixierungsstruktur (12) mit mindestens einer mechanischen Feder (15) gekoppelt ist, wobei
- die mechanische Feder (15) gespannt ist, wenn die Fixierungsstruktur (12) sich im kollabierten Zustand befindet, und
- die mechanische Feder (15) sich in der Ruhelage befindet, wenn die Fixierungsstruktur (12) sich im expandierten Zustand befindet.

12. Medizinisches Implantat (10) nach mindestens einem der Ansprüche 8 bis 11, wobei die Fixierungsstruktur (12) zumindest teilweise aus einem Formgedächtnismaterial, insbesondere einer Formgedächtnislegierung wie Nitinol, besteht.

13. Medizinisches Implantat (10) nach mindestens einem der Ansprüche 8 bis 12, wobei die Fassungseinheit (12.1, 12.4, 12.5) die Form eines Rings, einer Öse, einer Schlaufe oder eines Hakens aufweist.

14. System zur intravaskulären Implantation eines medizinischen Implantats (10) nach mindestens einem der vorhergehenden Ansprüche, mindestens aufweisend
- einen flexiblen Führungsdraht (14),
- eine Schiebeeinheit (23), umfassend einen flexiblen Schaft (23.1),
wobei das Implantat, der Führungsdraht (14) und die Schiebeeinheit (23) derart angeordnet sind, dass
- der Führungsdraht (14) mit dem ersten und zweiten Beförderungselement (13, 13') des Implantats zusammen wirkt, wobei bevorzugt das erste und zweite Beförderungselement (13, 13') jeweils ein Fassungsglied (13.1...13.6) aufweisen, welche den Führungsdraht (14) aufnehmen,
- der Führungsdraht (14) mindestens teilweise im Lumen des flexiblen Schafts (23.1) der Schiebeeinheit (23) angeordnet ist, wobei
- die Schiebeeinheit (23) dazu ausgestaltet ist, das medizinische Implantat (10) entlang des Führungsdrahts (14) zu schieben und/oder zu ziehen.

15. System nach Anspruch 14, wobei das medizinische Implantat (10) des Weiteren eine Fixierungsstruktur (12) mit mindestens zwei Fassungseinheiten (12.1, 12.4, 12.5) aufweist, wobei
- die Fixierungsstruktur (12) einen expandierten und einen kollabierten Zustand einnehmen kann, wobei
o die Fassungseinheiten (12.1, 12.4, 12.5) dazu ausgelegt sind, mit dem Führungsdraht (14) zusammen zu wirken, in dem der Führungsdraht (14) durch die Fassungseinheiten (12.1, 12.4, 12.5) aufgenommen wird, sodass sich die Fixierungsstruktur (12) im kollabierten Zustand befindet, und wobei
o die Fixierungsstruktur (12) dazu ausgelegt ist, in den expandierten Zustand überzugehen, sobald der Führungsdraht (14) nicht mit den Fassungseinheiten (12.1, 12.4, 12.5) zusammen wirkt.
